# EUROPEAN PATENT SPECIFICATION

(11) **EP 3 072 528 B1**
(45) Date of publication and mention of the grant of the patent: **05.07.2017**
(21) Application number: 15160959.1
(22) Date of filing: 26.03.2015
(51) Int. Cl.: A61K 47/00, A61K 47/30, A61K 9/16, A61K 9/50, A61K 31/437

(54) **COMPOSITION COMPRISING VEMURAFENIB AND CATIONIC COPOLYMER BASED ON METHACRYLATES**
ZUSAMMENSETZUNG MIT VEMURAFENIB UND KATIONISCHEM POLYMER AUF BASIS VON METHACRYLATEN
COMPOSITION COMPRENANT UN COPOLYMÈRE CATIONIQUE ET DU VEMURAFENIB BASÉ SUR DES MÉTHACRYLATES

(43) Date of publication of application: 28.09.2016
(73) Proprietor: ratiopharm GmbH, 89079 Ulm (DE)
(72) Inventor: Lehmann, Frank, 90233 Neu-Ulm (DE); Guserle, Richard, 89359 Kötz (DE); Albrecht, Wolfgang, 89075 Ulm (DE)
(74) Representative: Ter Meer Steinmeister & Partner

(56) References cited:
- WO-A1-2012/135750
- WO-A2-2010/114928
- NAVNIT SHAH ET AL: "Improved human bioavailability of vemurafenib, a practically insoluble drug, using an amorphous polymer-stabilized solid dispersion prepared by a solvent-controlled coprecipitation process", JOURNAL OF PHARMACEUTICAL SCIENCES, vol. 102, no. 3, 29 March 2013 (2013-03-29), pages 967-981, XP055112594, ISSN: 0022-3549, DOI: 10.1002/jps.23425
- RICHARD J BASTIN ET AL: "Salt Selection and Optimisation Procedures for Pharmaceutical New Chemical Entities", ORGANIC PROCESS RESEARCH AND DEVELOPMENT, AMERICAN CHEMICAL SOCIETY, US, vol. 4, no. 5, 19 July 2000 (2000-07-19), pages 427-435, XP008154792, ISSN: 1083-6160, DOI: 10.1021/OP000018U [retrieved on 2000-07-19]

## Description

### Background of the invention

The present invention relates to a premix comprising vemurafenib choline or vemurafenib as free form and cationic copolymer based on different methacrylates. Further, the present invention relates to dosage forms comprising said premix. The invention also relates to a process for producing the premix of the invention and to the corresponding process of producing an oral dosage form containing said premix. Further, the invention relates to a method for treating different types of cancer, such as colorectal cancer, ovarian carcinoma, thyroid cancer and malignant melanoma, in particular malignant melanoma, comprising administering the dosage form comprising the premix of the invention.

### Technical Background

Vemurafenib is an active pharmaceutical ingredient which is reported to be a potent and selective B-Raf kinase inhibitor. In particular, vemurafenib specifically inhibits the V600E mutation of B-Raf, which has been implicated in various tumours, for example colorectal cancer, ovarian carcinoma, thyroid cancer and malignant melanoma. It is reported that in xenoengrafted malignant melanomas B-Raf could be deactivated by vemurafenib and a regression of the tumor(s) could be achieved. Said mentioned V600E mutation in B-Raf is reported to be present in about 50% of the human malignant melanomas such that vemurafenib is effective to the genotype.

The IUPAC-name of "vemurafenib" is *N*-(3-{[5-(4-chlorophenyl)-1*H*-pyrrolo[2,3-b]pyridin-3-yl]carbonyl}-2,4-difluorophenyl)propane-1-sulfonamide. Alternatively "vemurafenib" can also be referred to as propane-1-sulfonic acid {3-[5-(4-chlorophenyl)-1H-pyrrolo[2,3-b]pyridine-3-carbonyl]-2,4-difluorophenyl}-amide. "Vemurafenib" is represented by the following chemical formula (1).

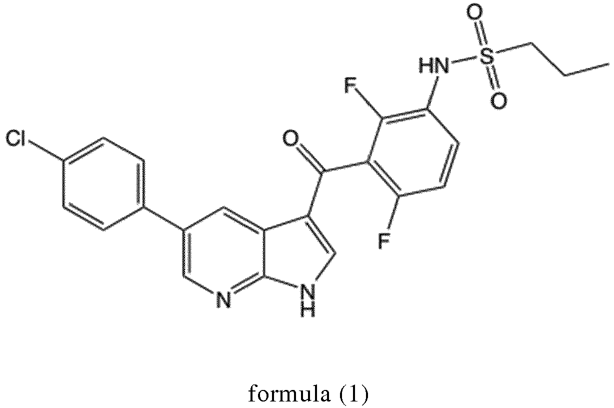

Vemurafenib base is reported to be a BCS IV compound and practically insoluble in aqueous and physiological solvents. Further, the HCl salt is reported to be better soluble.

WO 2012/135750 A1 relates to the provision of a combination of a) an AKT inhibitor or a pharmaceutically acceptable salt thereof and b) vemurafenib or a pharmaceutically acceptable salt thereof for the prophylactic or therapeutic treatment of a hyperproliferative disorder such as cancer.

WO 2010/114928 A2 relates to solid dispersions, solid molecular complexes, salts and crystalline polymorphs of vemurafenib. In particular WO 2010/114928 A2 describes solid dispersions comprising vemurafenib molecularly dispersed within a polymer matrix of an ionic polymer, wherein the weight ratio of active agent to polymer is preferably 1 : 4 to 1 : 1.5.

The teaching of WO 2010/114928 A2 was further developed in WO 2011/057974 A1. Said application discloses a method for manufacturing a solid dispersion and/or a solid dosage form containing vemurafenib and hydroxypropyl methyl cellulose acetate succinate, hereinafter referred to as HPMC-AS. Such an oral dosage form seems to be the marketed under the trade name "Zelboraf^{®}". Zelboraf^{®} is a tablet formulation comprising 240 mg vemurafenib as hydrochloric salt and 560 mg HPMC-AS as well as further excipients. Zelboraf^{®} is usually administered in a regime of 960 mg active agent (four tablets) twice a day.

However, a tablet having a weight of about 870 mg would be undesirably large in size and difficult to swallow. Patient compliance is expected to be rather poor. In addition, the patient compliance might be negatively affected by the high number of tablets (four tablets twice daily) to be taken each day. Further, Zelboraf^{®} is still improvable in view of bioavailability. In particular, the ratio of drug load/bioavailability might be improved. Additionally, Zelboraf^{®} is reported to cause constipation problems to some of the people to whom it was administered.

The article by Navnit Shah et al., "Improved human bioavailability of vemurafenib, a practically insoluble drug, using an amorphous polymer-stabilized solid dispersion prepared by a solvent-controlled coprecipitation process", JOURNAL OF PHARMACEUTICAL SCIENCES, vol. 102, no. 3, 29 March 2013, pages 967-981, deals with the improvement of the solubility of the practically insoluble drug vemurafenib by converting it into an amorphous-solid dispersion by using a solvent-controlled precipitation process.

The article by Richard J. Bastin et al., "Salt Selection and Optimisation Procedures for Pharmaceutical New Chemical Entities", ORGANIC PROCESS RESEARCH AND DEVELOPMENT, AMERICAN CHEMICAL SOCIETY, US, vol. 4, no. 5, 19 July 2000, pages 427-435, refers to the selection of an appropriate salt form for a new chemical entity.
Hence, it was an object of the present invention to overcome the above problems.

An object of the invention is to provide vemurafenib in a form ensuring good absorption properties in the gastrointestinal tract such as in the intestine. In particular, vemurafenib should be provided in a form capable of resulting in a so-called "nano-suspension", when dispersed under acidic condition, such as conditions like those existing in the stomach.

Moreover, an object of the present invention is to provide a dosage form, preferably a tablet, containing vemurafenib with dissolution properties similar or superior to the ones of Zelboraf^{®}, but having a higher drug-load, such that the dosage form is therefore smaller in size than Zelboraf^{®}.

Additionally or alternatively a dosage form containing vemurafenib with dissolution properties similar or equal to the ones of Zelboraf^{®} should be provided which can be administered in a smaller number of dosage forms per day.

### Summary of the Invention

According to the present invention, the above-mentioned technical problems can be solved by a premix comprising vemurafenib choline or vemurafenib as free form and cationic copolymer based on methacrylates. The premix can advantageously be processed into oral dosage forms of a size or number to be administered more comfortable to the patient.

Thus, the subject of the invention is a premix comprising
a) vemurafenib choline or vemurafenib as free form and
b) cationic copolymer based on methacrylates.

A further subject of the present invention is an oral dosage form comprising the premix of the invention and optionally further pharmaceutical excipient(s).

Another subject of the present invention is a method of preparing the premix according to the invention comprising the steps of
(i) providing vemurafenib choline or vemurafenib as free form and cationic copolymer based on methacrylates,
(ii) milling the mixture of step (i) and
(iii) optionally sieving the mixture of step (ii).

Further, the subject of the present invention relates to a process for preparing an oral dosage form comprising the steps of
(i) providing vemurafenib choline or vemurafenib as free form and cationic copolymers based on methacrylates,
(ii) milling the mixture of step (i),
(iii) optionally sieving the mixture of step (ii),
(iv) optionally adding further excipient(s) to the mixture of step (ii) or step (iii),
(v) optionally granulating the mixture of step (ii), step (iii) or step (iv) and
(vi) processing the mixture of step (ii), step (iii) or step (iv) or the granulates of step (v) into an oral dosage form.

The above-illustrated subjects of the present invention are alternative solutions to the above-outlined problems.

### Detailed Description of the Invention

The present invention concerns a premix comprising vemurafenib choline or vemurafenib as free form and cationic copolymer based on methacrylates, wherein the components are preferably present in a specific weight ratio. In a particular preferred embodiment vemurafenib choline is used.

Vemurafenib choline can be represented by the following chemical formula (2). It can be prepared according to WO 2014/008270.

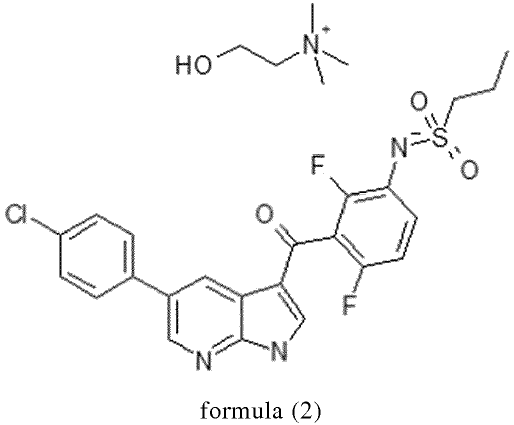

"Vemurafenib" in free form can be illustrated by to the above formula (1). That means, the free form does not encompass a salt form. Further, preferably the free form does not encompass a hydrated/solvated form.

Vemurafenib as free form preferably corresponds to vemurafenib as free base.

In a particularly preferred embodiment the premix of the present invention as well as the oral dosage form of the present invention comprise vemurafenib choline or vemurafenib as free form as the sole pharmaceutical active agent. Particularly preferred it comprises vemurafenib choline as the sole pharmaceutical active agent.

In an alternative embodiment the composition of the present invention as well as the oral dosage form of the present invention can comprise vemurafenib choline or vemurafenib as free form in combination with further pharmaceutical active agent(s).

A further component in the premix of the present invention is a cationic copolymer based on methacrylates.

In a preferred embodiment the cationic copolymer based on methacrylates is water-soluble at a pH-value up to 5.0. At a pH above 5.0 the cationic copolymer based on methacrylates can preferably be water-insoluble.

In a preferred embodiment the cationic copolymer based on methacrylates can have an alkali value from 75 to 300 mg KOH/g polymer, more preferably 100 to 275 mg KOH/g polymer, in particular 125 to 250 mg KOH/g polymer.

A cationic copolymer based on methacrylates is a copolymer with a cationic character, wherein the cationic copolymer comprises different acrylic monomer units. Preferably, the copolymer is based on 2-dimethylaminomethyl methacrylate and neutral alkylmethacrylates, wherein alkyl is an alkyl containing 1 to 6 carbon atoms. In particular neutral alkylmethacrylates are methyl methacrylate and butyl methacrylate.

A preferred cationic copolymer is based on dimethylaminomethyl methacrylate, methyl methacrylate and butyl methacrylate. A particular preferred cationic copolymer is poly(butyl methacrylate-co-(2-dimethylaminoethyl)methacrylate-co-methyl methacrylate) wherein the ratio is about 1:2:1.

The cationic polymer can be illustrated by the following simplified formula

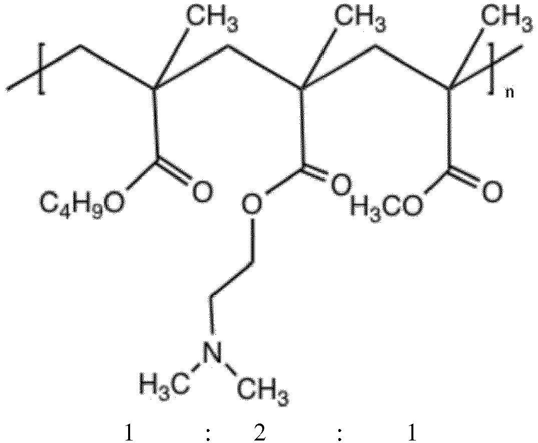

In a preferred embodiment poly(butyl methacrylate-co-(2-dimethyl-aminoethyl)methacrylate-co-methyl methacrylate can have a weight average molecular weight ranging from 10,000 to 100,000 g/mol, preferably from 15,000 to 85,000 g/mol, more preferably from 25,000 to 60,000 g/mol. The weight average molecular weight can preferably be determined by gel electrophoresis.

Further, poly(butyl methacrylate-co-(2-dimethylaminoethyl)methacrylate-co-methyl methacrylate preferably may have a glass transition temperature (Tg) of 25°C to 60°C, more preferably of 28°C to 57°C, in particular of 32°C to 54°C.

In the present invention the term "glass transition temperature" (Tg) describes the temperature at which amorphous or partially crystalline polymer change from the solid state to the liquid state. In the process a distinct change in physical parameters, e.g. hardness and elasticity, occurs. Beneath the Tg a polymer is usually glassy and hard, whereas above the Tg it changes into a rubber-like to viscous state. The glass transition temperature is determined in the context of this invention by means of differential scanning calorimetry (DSC).

For this purpose, a Mettler Toledo^{®} DSC 1 apparatus can be used. The work is performed at a heating rate of 1-20°C/min, preferably 10°C/min, and at a cooling rate of 5°C to 50°C/min, preferably 50°C/min.

In a particularly preferred embodiment of the invention the cationic polymer based on methacrylates can be basic butylated methacrylate copolymer according to Ph.Eur.

In a preferred embodiment the premix of the present invention can preferably comprise 33 to 50 wt%, more preferably 35 to 47 wt% and more preferably 37 to 45 wt% cationic polymer based on methacrylates, based on the total weight of the premix.

In a preferred embodiment the cationic copolymer based on methacrylates does not comprise a quaternary ammonium group.

The premix of the present invention can preferably contain vemurafenib choline or vemurafenib as free form in non-crystalline form.

The term "non-crystalline" can be used in the context of this invention to designate a compound in which the components (atoms, ions or molecules) do not exhibit a periodic arrangement over a great range (=long-range order) as usually known from crystalline substances. Consequently, in contrast to crystalline substances, X-ray diffraction performed on non-crystalline substances does not reveal clearly defined interferences for them, but normally only a few diffuse interferences with small diffraction angles.

In a preferred embodiment non-crystalline vemurafenib choline or vemurafenib as free form relates to amorphous vemurafenib choline or vemurafenib as free form. The term "amorphous" is used in the context of this invention to designate the state of solid substances in which the components (atoms, ions or molecules, for example in case of amorphous vemurafenib (the corresponding molecules) do not exhibit any periodic arrangement over a great range (=long-range order). In amorphous substances, the components are usually not arranged in a totally disordered fashion and completely randomly, but are rather distributed in such a way that a certain regularity and similarity to the crystalline state can be observed with regard to the distance from and orientation towards their closest neighbours (=short-range order). Amorphous substances consequently preferably possess a short-range order, but no long-range order. In contrast to anisotropic crystals, solid amorphous substances are isotropic. The amorphous substances can be distinguished experimentally from crystalline substances by means of X-ray diffraction, where the amorphous substances do not reveal clearly defined interferences as mentioned above.

In the context of this invention, the expression "amorphous vemurafenib choline or vemurafenib as free form" preferably refers to a substance which consists of amorphous vemurafenib choline or vemurafenib as free form. Alternatively, "amorphous vemurafenib choline or vemurafenib as free form" may also contain small amounts of crystalline vemurafenib choline or vemurafenib as free form components, provided that no defined interferences in X-ray diffraction can be detected. A mixture containing 85 to 99.99% by weight amorphous vemurafenib choline or vemurafenib as free form and 0.01 to 15% crystalline vemurafenib choline or vemurafenib as free form is preferred, more preferably 95 to 99.9% by weight amorphous vemurafenib choline or vemurafenib as free form and 0.1 to 5% crystalline vemurafenib choline or vemurafenib as free form. In a particularly preferred embodiment amorphous vemurafenib choline or vemurafenib as free form does not contain crystalline vemurafenib choline or vemurafenib as free form at all. The crystalline proportion is determined by means of quantitative X-ray diffractometry according to the method of Hermans and Weidinger, wherein porcelain powder can be added as reference.

In an alternatively preferred embodiment non-crystalline vemurafenib choline or vemurafenib as free form relates to vemurafenib choline or vemurafenib as free form in the form of a solid solution. The term "solid solution" is to be understood in the context of this invention as meaning that non-crystalline vemurafenib choline or vemurafenib as free form is distributed in a molecularly disperse manner in matrix of cationic copolymer based on methacrylates which is present in a solid aggregate state at 25°C and a pressure of 101 kPa.

It is preferable that in this alternative embodiment, the premix of the invention (containing non-crystalline vemurafenib choline or vemurafenib as free form in the form of a solid solution) contains substantially no crystalline or amorphous vemurafenib choline or vemurafenib as free form. In particular, the premix of the invention contains less than 15 wt%, more preferably less than 5 wt% of crystalline or amorphous vemurafenib choline or vemurafenib as free form, based on the total weight of the non-crystalline vemurafenib choline or vemurafenib as free form present in the premix.

Further, it is preferable for "molecularly disperse" to be understood as meaning that X-ray diffraction analysis of the non-crystalline vemurafenib choline or vemurafenib as free form contained in the embodiments of the invention does not reveal any clearly defined interference patterns, but at most only a few diffuse interferences with small diffraction angles.

In the context of this alternative embodiment of this invention, the solid solution of non-crystalline vemurafenib choline or vemurafenib as free form of the invention is present in a stabilised form, namely in the form of a premix containing molecularly disperse non-crystalline vemurafenib choline or vemurafenib as free form and cationic copolymer of methacrylates (as a matrix material). In particular, the premix of the invention consists substantially of molecularly disperse non-crystalline vemurafenib choline or vemurafenib as free form and cationic copolymer of methacrylates. The expression "substantially" in this case indicates that small amounts of solvent etc. may also still be contained where applicable.

In a preferred embodiment the premix of the present invention can have a weight ratio of vemurafenib choline to cationic polymer based on methacrylates from 1:4 to 4:1, preferably from 1:3 to 3:1, more preferably from 1:1 to 2:1, even more preferably from 1.01:1 to 1.4:1, in particular from 1.02:1 to 1.25:1, especially from 1.03:1 to 1.15:1.

The premix of the present invention can preferably have a weight ratio of vemurafenib as free form to cationic polymer based on methacrylates from 1:4 to 4:1, preferably from 1:3 to 3:1, more preferably from 1:1 to 2:1, even more preferably from 1.01:1 to 1.4:1, in particular from 1.02:1 to 1.25:1, especially from 1.03:1 to 1.15:1.

It turned out that with the above ratio it can be particularly ensured that the complete amount of vemurafenib choline or vemurafenib as free form, preferably vemurafenib choline, remains in a non-crystalline state and does reconvert to a crystalline substance.

In a particular preferred embodiment the premix can preferably contain vemurafenib as free form in an amount of 240 mg or 480 mg.

In a preferred embodiment of the invention the premix can preferably contain vemurafenib choline in an amount from 280 mg to 620 mg, preferably 315 mg to 540 mg, more preferably 380 to 475 mg.

In a preferred embodiment the premix of the present invention can preferably comprise the following amounts of components:
a) vemurafenib choline in an amount from 280 mg to 620 mg, preferably 290 mg, and
b) 110 to 510 mg cationic copolymer based on methacrylates.

In an alternative preferred embodiment the premix of the present invention can preferably comprise the following amounts of components:
a) vemurafenib as free form in an amount from 230 mg to 520 mg, preferably 240 mg, and
b) 110 to 510 mg cationic copolymer based on methacrylates.

In a preferred embodiment the premix of the invention can have a particle size of 0.1 to 100 µm, preferably 1 to 50 µm, more preferably 2 to 40 µm, in particular 5 to 30 µm, especially 6 to 20 µm. The particle size can be determined by means of the microscope "Leica DM2500P", wherein the premix was dispersed in Halocarbon oil 700. When determining the particle size by microscope the "longest dimension" of the particle is measured.

It turned out that the present premix, when introduced into 0.01 N HCl and stirred, preferably for one hour, forms a milky suspension which can be regarded as nanosuspension. The suspension is unexpectedly stable.

The particles within the nanosuspension can preferably have a mean particle size D₅₀ of 200 nm to 1 µm, more preferably 250 to 850 nm, even more preferably 300 to 750 nm, in particular 350 to 600 nm.

The mean particle size (in the nanosuspension) can refer to the D₅₀ of the particle size distribution. The average particle size can be determined by means of laser diffractometry. In particular, a Malvern Zetasizer Nano Series Nano-ZS90 can be used to determine the size (wet measurement; duration: 50 sec.; disposable micro cuvette; Temp.: 25.1°C; suspension of the sample in 0.1 M HCl further diluted 1:10 with water; dispersant: water, dispersant RI: 1.330; Viscosity (cP): 0.8872).

The premix of the present invention can be applied in form of an oral dosage form, in particular in form of a solid oral dosage form. Thus, another object of the present invention is a solid oral dosage form comprising the premix according to the present invention.

In a preferred embodiment the present oral dosage form can preferably comprise further pharmaceutical excipient(s).

The pharmaceutical excipients are excipients with which the person skilled in the art is familiar, such as those described in the European Pharmacopoeia (Ph.Eur.) and/or in the US Pharmacopoeia (USP).

In a preferred embodiment of the present invention the oral dosage form can further comprise one or more excipients(s) selected from surfactants (c), fillers (d), binders (e), disintegrants (f), lubricants (g) and glidants (h).

Surfactants (c) can be regarded as substances lowering the interfacial tension between two phases, thus enabling or supporting the formation of dispersions or working as a solubilizer. Common surfactants are alkylsulfates (for example sodium lauryl sulfate), alkyltrimethylammonium salts, alcohol ethoxylates and the like. Surfactants can be used in an amount of 0 to 2 wt%, preferably of 0.1 to 1.5 wt%, based on the total weight of the oral dosage form.

Fillers (d) or diluents can be used to increase the bulk volume and weight of a low-dose drug to a limit at which a pharmaceutical dosage form can be formed. Fillers should fulfil several requirements, such as being chemically inert, non-hygroscopic, biocompatible, easily processable and possessing good biopharmaceutical properties. Examples of fillers are lactose, sucrose, glucose, mannitol, calcium carbonate, cellulose and others. Fillers can be used in an amount of 0 to 25 wt%, preferably 1 to 20 wt%, based on the total weight of the dosage form.

It is particularly preferred that the oral dosage form of the present invention does not contain a filler.

Binders (e) may be added to the pharmaceutical formulation in order to ensure that oral dosage forms, preferably tablets, can be formed with the required mechanical strength. The binder can, for example, be starch, polyvinyl pyrrolidone or cellulose derivatives such as hydroxypropyl cellulose. Binders can be present in an amount of 0 to 10 wt%, preferably 0.2 to 8 wt%, more preferably 0.4 to 5 wt%, based on the total weight of the pharmaceutical formulation.

Disintegrants (f) are compounds which enhance the ability of the dosage form, preferably the ability of the tablet to break into smaller fragments when in contact with a liquid, preferably water. Preferred disintegrants are sodium carboxymethyl starch, cross-linked polyvinyl pyrrolidone (crospovidone), sodium carboxymethyl glycolate, swelling polysaccharide, for example soy polysaccharide, carrageenan, agar, pectin, starch and derivatives thereof or protein, for example formaldehyde-casein, sodium bicarbonate or mixtures thereof. More preferred are sodium carboxymethyl cellulose and cross-linked polyvinyl pyrrolidone (crospovidone). Disintegrants can be used in an amount of 0 to 15 wt%, preferably of 1 to 12 wt%, more preferably 2.5 to 8 wt%, based on the total weight of the dosage form.

The function of lubricants (g) is reported to ensure that tablet formation and ejection can occur with low friction between the solid and the die wall. Further, lubricants can generally increase the powder flowability. The lubricant is preferably a stearate or fatty acid, more preferably an earth alkali metal stearate, such as magnesium stearate. The lubricant is suitably present in an amount of 0 to 2 wt%, preferably of about 0.1 to 1.0 wt%, based on the total weight of the dosage form.

Glidants (h) can also be used to improve the flowability. Traditionally, talc was used as glidant, but is nowadays nearly fully replaced by colloidal silica (for example Aerosil^{®}). Preferably, the glidant can be present in an amount of 0 to 3 wt%, more preferably 0.1 to 2.5 wt%, in particular 0.25 to 2.0 wt%, based on the total weight of the dosage form.

It lies in the nature of pharmaceutical excipients that they sometimes can perform more than one function in a pharmaceutical formulation. In this regard it is generally noted that due to the nature of pharmaceutical excipients it cannot be excluded that a certain compound meets the requirements of more than one of components (c) to (h). However, in order to enable an unambiguous distinction, it is preferred in the present application that one and the same pharmaceutical compound can only function as one of the compounds (c) to (h). For example, if microcrystalline cellulose functions as filler (d), it cannot additionally function as disintegrant (f), even though microcrystalline cellulose also exhibits a certain disintegrating effect.

The oral dosage form of the present invention can preferably comprise
40 to 70 wt% vemurafenib choline or vemurafenib as free form, preferably 43 to 68 wt% vemurafenib choline or vemurafenib as free form,
25 to 45 wt% cationic copolymer based on methacrylates, preferably 28 to 42 wt% cationic copolymer based on methacrylates,
0 to 2 wt% surfactant, preferably 0.1 to 1.5 wt% surfactant,
0 to 25 wt% filler, preferably 1 to 20 wt% filler,
0 to 10 wt% binder, preferably 0.2 to 8 wt% binder,
0 to 15 wt% disintegrant, preferably 1 to 12 wt% disintegrant,
0 to 2 wt% lubricant, preferably 0.1 to 1.0 wt% lubricant and
0 to 3 wt% glidant, preferably 0.1 to 2.5wt% glidant,
based on the total weight of the oral dosage form.

In a preferred embodiment of the invention the dosage form of the present invention can preferably comprise vemurafenib choline in an amount of 270 mg to 620 mg.

In a more preferred embodiment the dosage form of the present invention can preferably comprise vemurafenib choline in an amount of 270 to 305 mg, even more preferably 287 to 299 mg, in particular about 291 mg.

In an alternative more preferred embodiment the dosage form of the present invention can preferably comprise vemurafenib choline in an amount of 540 to 610 mg, even more preferably 574 to 598 mg, in particular about 582 mg.
In a preferred embodiment the total weight of the dosage form is 400 to 750 mg, preferably 450 to 750 mg, in case when 270 to 305 mg vemurafenib choline are contained in the dosage form. In an alternative preferred embodiment the total weight of the dosage form is from 800 to 1450 mg, preferably from 900 to 1350 mg, in case when 540 to 610 mg vemurafenib choline are contained in the dosage form.

In a still further embodiment of the present invention the oral dosage form can be a solid oral dosage form, preferably a capsule or a tablet, more preferably a tablet, in particular for peroral use. Alternatively, the solid oral dosage form can be filled as powder or granulate into devices like sachets or stick-packs.

The present invention further relates to a method for producing a premix according to the invention. Hence, a further subject of the present invention is a method for preparing a premix comprising vemurafenib choline or vemurafenib as free form and cationic copolymer based on methacrylates comprising the steps of
(i) providing vemurafenib choline or vemurafenib as free form and cationic copolymer based on methacrylates,
(ii) milling the mixture from step (i) and
(iii) optionally sieving the mixture from step (ii).

Generally, the comments made above for vemurafenib choline or vemurafenib as free form and cationic copolymer based on methacrylates can also apply to the method of the present invention.

In step (i) vemurafenib choline or vemurafenib as free form and cationic copolymer based on methacrylates are provided. Vemurafenib choline or vemurafenib as free form can preferably be provided in a crystalline form. Further, vemurafenib choline or vemurafenib as free form can be provided in micronized form. The step of micronisation can preferably be carried out by milling, such as in an air jet mill. Preferably, the average particle size D₅₀ of vemurafenib choline or vemurafenib as free form is from 1 to 25 µm, preferably from 2 to 20 µm and can be determined as described above. Optionally, vemurafenib choline or vemurafenib as free form and cationic copolymer based on methacrylates can be blended in order to provide a composition having a homogenous distribution of vemurafenib within the resulting blend comprising vemurafenib choline or vemurafenib as free form and cationic copolymer based on methacrylates. Blending can be carried out with conventional mixing devices, e.g. in a free-fall mixer like Turbula^{®} T10B (Bachofen AG, Switzerland). Blending can be carried out e.g. for 1 minute to 30 minutes, preferably for 2 minutes to less than 10 minutes.

In an alternative embodiment, step (i) can include dissolving, preferably completely dissolving, vemurafenib choline or vemurafenib as free form and cationic copolymer based on methacrylates in a solvent. Dissolving, preferably completely dissolving, can preferably be carried out under stirring, for example for 5 to 15 minutes. It is further preferred that dissolving can preferably carried out at 23°C (room temperature). Suitable solvents can be alcohols with 1 to 4 carbon atoms, preferably methanol or ethanol, tetrahydrofuran and mixtures thereof. Particularly methanol is used. Subsequently, the solvent can be evaporated, preferably completely evaporated. The obtained solid can preferably be dried at 23°C until constant weight.

In step (ii) the milling of the mixture from step (i) can preferably be performed in conventional milling apparatuses, such as in a ball mill, air jet mill, pin mill, classifier mill, cross beater mill, disk mill, mortar grinder or a rotor mill. A ball mill is preferably used.

The milling time is preferably 5 minutes to 6 hours, preferably 10 minutes to 4 hours, more preferably 15 minutes to 2 hours.

In a preferred embodiment the milling conditions in step (ii) can preferably be selected such that the vemurafenib choline or vemurafenib as free form is in a non-crystalline form, i.e. that the XRD of the milled mixtures does not reveal any clearly defined interference patterns of vemurafenib choline or vemurafenib as free form, but at most only a few diffuse interferences with small diffraction angles.

It is preferred that the optional step (iii) of sieving the mixture of step (ii) can be carried out with a sieve having a mesh size of 25 to 1000 µm, preferably 50 to 800 µm, especially 100 to 600 µm.

In a preferred embodiment the present premix is not in form of a co-precipitate. In particular, the present premix is not prepared by dissolving, preferably completely dissolving, vemurafenib choline or vemurafenib as free form and cationic polymer based on methacrylates in a solvent or mixtures of solvents and forming the co-precipitate by the addition of one or more anti-solvents, wherein subsequently the co-precipitate can be isolated.

Further, the subject of the present invention relates to a method for preparing the oral dosage form of the invention comprising the steps:
(i) providing vemurafenib choline or vemurafenib as free form and cationic copolymer based on methacrylates,
(ii) milling the mixture from step (i),
(iii) optionally sieving the mixture from step (ii),
(iv) optionally adding further excipient(s) to the mixture of step (ii) or step (iii),
(v) optionally granulating the mixture of step (ii), step (iii) or step (iv) and
(vi) processing the mixture of step (ii), step (iii), or step (iv) or the granulates of step (v) into an oral dosage form.

In steps (i) to (iii) a premix according to the present invention is provided, i.e. all the above process steps (i), (ii) and (iii) leading to the present premix also apply to the process for preparing the present oral dosage form.

In step (iv) additional further excipient(s) and/or further pharmaceutically active agent can optionally be added to the mixture of step (ii) or step (iii). During or after the addition of the optional excipients and/or further pharmaceutically active agent the resulting mixture can preferably be blended. Blending can be carried out with the conventional mixing devices as described above. The excipients can preferably be selected from the excipients (c) to (h) as described above.

In step (v) the mixture of step (ii), step (iii) or step (iv) can be optionally granulated.

"Granulating" is generally understood to mean the formation of relatively coarse or granular aggregate material as a powder by assembling and/or aggregating finer powder particles (agglomerate formation or build-up granulation) and/or the formation of finer granules by breaking up coarser aggregates (disintegration or break-down granulation).

Granulation can conventionally mean wet or dry granulation.

Dry granulation, which is preferred, is generally carried out by using pressure or temperature. In a preferred embodiment of the invention, granulating the mixture from step (ii), step (iii) or step (iv) can be performed for example by "slugging" using a large heavy-duty rotary press and breaking up the slugs into granulates with a hammer mill or by roller compaction using for example roller compactors by Powtec or Alexanderwerk. The granulates are then optionally screened.

In step (vi) the mixture of step (ii), step (iii) or step (iv) or the granulates of step (v) can be processed into a solid oral dosage form. Processing the mixture of step (ii), step (iii) or step (iv) or the granulates from step (v) into a solid oral dosage form can preferably comprise filling said mixture of step (ii), step (iii) or step (iv) or said granulates from step (v) into capsules, preferably hard gelatine capsules.

More preferred, processing the mixture of step (ii), step (iii) or step (iv) or the granulates from step (v) into tablets can be carried out by compressing said formulation on a rotary press, e.g. on a Fette^{®} (Fette GmbH, Germany) or a Riva^{®} piccola (Riva, Argentina). The main compression force can range from 1 to 50 kN, preferably from 3 to 40 kN. The resulting tablets can have a hardness of 30 to 400 N, more preferred of 50 to 250 N, particularly preferably of 30 to 180 N, especially 40 to 150 N, wherein the hardness can be measured according to Ph.Eur. 6.0, Chapter 2.9.8. For the optional filling of the formulation into capsules dependent dosing systems (for example an auger) or preferably independent dosing systems (for example MG2, Matic (IMA)) can be used.

Further, the dosage form, preferably the tablet, of the invention preferably has a content uniformity of 90 to 110%, i.e. a content of active agent(s) which lies within the concentration of 90 to 110%, preferably 95 to 105%, especially preferred from 98 to 102% of the average content of the active agents(s). The "content uniformity" is determined with a test in accordance with Ph. Eur., 6.0, Chapter 2.9.6. According to that test, the content of the active agent of each individual tablet out of 20 tablets must lie between 90 and 110%, preferably between 95 and 105%, especially between 98 and 102% of the average content of the active agent(s). Therefore, the content of the active drugs in each tablet of the invention differs from the average content of the active agent by at most 10%, preferably at most 5% and especially at most 2%.

In addition, the resulting tablet preferably has a friability of less than 5%, particularly preferably less than 2%, especially less than 1%. The friability is determined in accordance with Ph. Eur., 6.0, Chapter 2.9.7. The friability of tablets generally refers to tablets without coating.

The oral dosage form the invention may be a peroral tablet which can be swallowed unchewed. The tablet can preferably be film coated.

Generally, film coatings that do not affect the release of the active agent(s) and film coatings affecting the release of the active agent(s) can be employed with tablets according to invention. The film coatings that do not affect the release of the active agent(s) are preferred.

Preferred examples of film coatings which do not affect the release of the active ingredient can be those including poly(meth)acrylate, methylcellulose (MC), hydroxypropyl methylcellulose (HPMC), hydroxypropyl cellulose (HPC), hydroxyethyl cellulose (HEC), polyvinylpyrrolidone (PVP) and mixtures thereof. These polymers can have a weight-average molecular weight of 10,000 to 150,000 g/mol.

In an alternative preferred embodiment, the film coating can affect the release of the active agent. Examples for film coatings affecting the release of the active agent are gastric juice-resistant film coatings and retard coatings.

In a preferred embodiment the film can have a thickness of 2 µm to 150 µm, preferably from 10 to 100 µm, more preferably from 20 to 60 µm.

In a preferred embodiment the dosage form of the invention is for modified release. In that case the release profile of the pharmaceutical formulation, preferably of the tablet, according to USP method (USP paddle apparatus, 900 ml test medium, in phosphate buffer at pH 6.8 and 37°C, 100 rpm) after 2 hours indicates a content release of 0 to 90%, preferably of 10 to 80%, further preferably 15 to 75%, more preferably 20 to 50% and particularly of 25 to 40%.

In a more preferred embodiment of the invention the dosage form is for immediate release. In that case the release profile of the pharmaceutical formulation, preferably of the tablet, according to USP method (USP paddle apparatus, 900 ml test medium, in phosphate buffer at pH 6.8 and 37°C, 100 rpm) after 15 minutes indicates a content release of at least 70%, preferably at least 80%, especially from 85 to 99 %.

Further, the invention relates to a premix of the invention or the dosage form of the invention for use in the treatment of cancer, preferably colorectal cancer, ovarian carcinoma, thyroid cancer and/or malignant melanoma. For the pharmaceutical composition used in the before-mentioned treatment the same applies as to the premix and the oral dosage form as described above in the text.

### Experimental Part

### Analytical Methods

The samples were examined by X-ray powder diffraction.

### X-Ray Powder Diffraction

The measurements were performed as follows: The samples were measured on a D8 Advance powder X-ray diffractometer (Bruker-AXS, Karlsruhe, Germany) in a PMMA sample holder rotating at 20 rpm during the measurement (Bragg-Brentano geometry). Further conditions for the measurements are summarized below. The raw data were analysed with the program EVA (Bruker-AXS, Karlsruhe, Germany).

| | |
|---|---|
| Detector: | Vantec-1 3°2θ |
| Radiation: | Cu K_{α} (1.5418Å) |
| Monochromator: | None |
| Second ß filter: | Ni 0.1 mm |
| Start angle: | 2° |
| End angle: | 55° |
| Measurement time: | 11 min |
| Step: | 0.016° 2θ |

### Solubility

The solubility of vemurafenib choline or vemurafenib as free form is determined in 50 nM potassium phosphate buffer, pH 6.5, 2% TPGS (d-alpha tocopheryl glycol 1000 succinate).

### Solubility (S₁):

The following procedure was used for rapidly and reliably determining the properties of the below Vemurafenib-preparations. The samples were mixed on a Vortex mixer for a period of 10 minutes. In addition, the samples were subsequently subjected to stirring for 1 h at 500 rpm. Thereafter, the solubility was determined in the above-mentioned medium.

### Solubility with HCl-pretreatment (S₂):

In order to simulate the exposure of samples in the gastro-intestinal tract, the samples were subjected to stirring at 500 rpm for one hour in gastric-like pH conditions (0.1 N HCl). Thereafter, the solubility either of the suspension or of the precipitate (after isolation) was determined in the above-mentioned medium.

### EXAMPLES

### Example 1

500 mg of crystalline vemurafenib choline and 500 mg of basic butylated methacrylate copolymer were milled in a "Fritsch pulverisette" for 1 hour at 400 rpm with 10 balls, each having a diameter of 10 mm.

### Example 2

1 g crystalline vemurafenib choline and 1 g of basic butylated methacrylate copolymer were dissolved in 20 mL methanol at 23°C (RT). The mixture was stirred for 10 minutes and subsequently evaporated to dryness. The obtained solid was dried under ambient conditions (23°C (RT)/no vacuum) for 16 h. The product was milled in a "Fritsch pulverisette" for 1 hour at 400 rpm with 10 balls, each having a diameter of 10 mm.

### Example 3

1 g crystalline vemurafenib choline salt and 0.5 g of basic butylated methacrylate copolymer were dissolved in 20 mL methanol at RT. The mixture was stirred for 10 minutes and subsequently evaporated to dryness. The obtained solid was dried under ambient conditions (RT/no vacuum) for 16 h. The product was milled in a "Fritsch pulverisette" for 1 hour at 400 rpm with 10 balls, each having a diameter of 10 mm.

### Reference Example 1

A solid dispersion comprising vemurafenib free base and HPMC-AS in a weight ratio of 3:7 was prepared according to Examples 1 to 5 of WO 2011/057974.

### RESULTS

The solubility of the samples was determined according to the precedure as mentioned above. The results are specified in Table A.

**Table A**

| **Sample** | **sample weight [mg]** | **solvent [µl]** | **S₁ [mg/ml]** | **S₂ [mg/ml]** |
|---|---|---|---|---|
| Example 2 | 8.46 | 1000 | 0.689 | 4.396 |
| Example 3 | 12.72 | 1000 | 0.378 | 3.990 |
| Reference Example 1 | 25.35 | 1000 | 1.611 | 1.524 |

As can be seen, the solubility S₂ of the present Examples is significantly higher than the one of the Reference Example, which corresponds to the market product Zelboraf^{®}. Since compared to Reference Example 1 a proportionally small amount of basic butylated methacrylate copolymer is used with regard to the active agent, the present premix allows formulating a dosage form having a higher drug load or alternatively a dosage form with the same content of active agent but being significantly smaller in size.

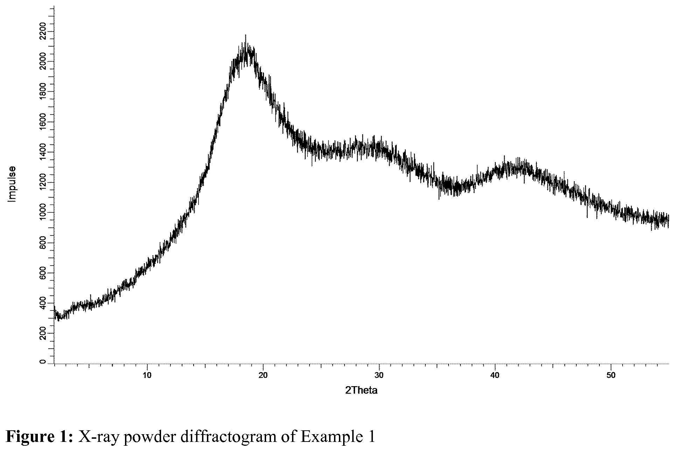

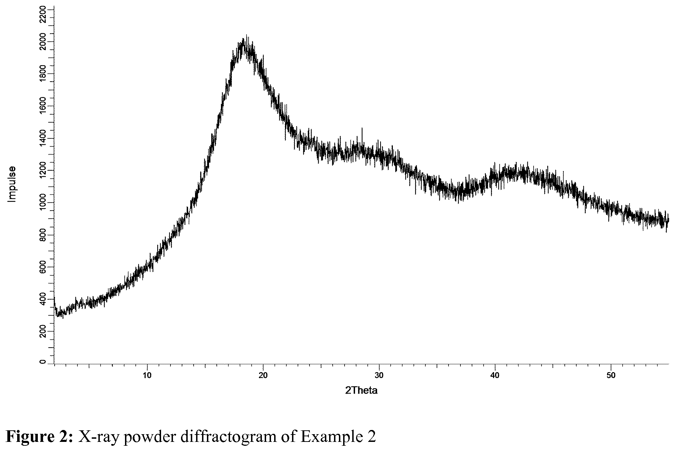

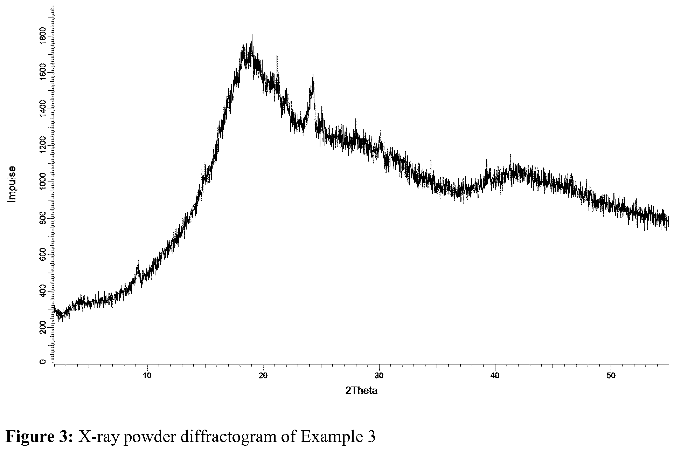

Further, X-ray powder diffraction was conducted for Example 1, Example 2, and Example 3. As can be seen from Figures 1, 2 and 3 the corresponding X-ray powder diffractograms do not show any defined interferences. Thus, in the present premix vemurafenib choline or vemurafenib as free form is present mainly in amorphous form.

## Claims

1. Premix comprising
a) vemurafenib choline or vemurafenib as free form, and
b) cationic copolymer based on methacrylates.

2. Premix according to claim 1, wherein the vemurafenib choline or vemurafenib as free form is present in a non-crystalline form.

3. Premix according to claim 1 or 2, wherein the weight ratio of vemurafenib choline to cationic copolymer based on methacrylates is from 1:4 to 4:1.

4. Premix according to claim 1 or 2, wherein the weight ratio of vemurafenib as free form to cationic copolymer based on methacrylates is from 1:4 to 4:1.

5. Premix according to any one of claims 1 to 4, wherein the premix has a particle size of 0.1 to 100 µm, determined by means of the microscope "Leica DM2500P".

6. Oral dosage form comprising the premix according to any one of claims 1 to 5.

7. Oral dosage form according to claim 6, wherein the dosage form comprises
40 to 70 wt% vemurafenib choline or vemurafenib as free form,
25 to 45 wt% cationic copolymer based on methacrylates as premix and
0 to 2 wt% surfactant
0 to 25 wt% filler,
0 to 10 wt% binder,
0 to 15 wt% disintegrant,
0 to 2 wt% lubricant and
0 to 3 wt% glidant,
based on the total weight of the oral dosage form.

8. Oral dosage form according to claim 6 or 7, wherein the dosage form comprises 270 to 305 mg vemurafenib choline or 540 to 610 mg vemurafenib choline.

9. Oral dosage form according to any one of claims 6 to 8, wherein the dosage form has a total weight of 400 to 750 mg or of 800 to 1450 mg.

10. Oral dosage form according to any one of claims 6 to 9, wherein the content release of the dosage form after 15 minutes is 70 % to 100 % when measured according to USP paddle apparatus type II, 900 ml, phosphate buffer at pH 6.8 and 37°C at 100 rpm.

11. Method for preparing the premix according to claims 1 to 5 comprising the steps of
(i) providing vemurafenib choline or vemurafenib as free form and cationic copolymer based on methacrylates, (ii) milling the mixture from step (i) and
(iii) optionally sieving the mixture from step (ii)

12. Method according to claim 11, wherein the milling conditions in step (ii) are selected such that the vemurafenib choline or vemurafenib as free form is obtained after milling in a non-crystalline form.

13. Method for preparing an oral dosage form according to any one of claims 6 to 10 comprising the steps of
(i) providing vemurafenib choline or vemurafenib as free form and cationic copolymer based on methacrylates,
(ii) milling the mixture from step (i),
(iii) optionally sieving the mixture from step (ii),
(iv) optionally adding further excipient(s) to the mixture of step (ii) or step (iii),
(v) optionally granulating the mixture of step (ii), step (iii) or step (iv) and
(vi) processing the mixture of step (ii), step (iii) or step (iv) or the granulates of step (v) into an oral dosage form.

14. Dosage form according to any one of claim 6 to 10 for use in the treatment of cancer, preferably colorectal cancer, ovarian carcinoma, thyroid cancer and/or malignant melanoma.

## Patentansprüche

1. Vormischung enthaltend,
a) Vemurafenib-Cholin oder Vemurafenib als freie Form, und
b) kationisches, auf Methacrylaten basierendes Copolymer.

2. Vormischung gemäß Anspruch 1, wobei das Vemurafenib-Cholin oder Vemurafenib als freie Form in einer nicht-kristallinen Form vorliegt.

3. Vormischung gemäß Anspruch 1 oder 2, wobei das Gewichtsverhältnis von Vemurafenib-Cholin zu kationischem, auf Methacrylaten basierendem Copolymer von 1:4 bis 4:1 ist.

4. Vormischung gemäß Anspruch 1 oder 2, wobei das Gewichtsverhältnis von Vemurafenib als freie Form zu kationischem, auf Methacrylaten basierendem Copolymer von 1:4 bis 4:1 ist.

5. Vormischung gemäß einem der Ansprüche 1 bis 4, wobei die Vormischung eine Teilchengröße von 0.1 bis 100 µm aufweist, bestimmt mittels des Mikroskops "Leica DM2500P".

6. Orale Darreichungsform, welche die Vormischung gemäß einem der Ansprüche 1 bis 5 enthält.

7. Orale Darreichungsform gemäß Anspruch 6, wobei die Darreichungsform umfasst
40 bis 70 Gew.% Vemurafenib-Cholin oder Vemurafenib als freie Form,
25 bis 45 Gew.% kationisches, auf Methacrylaten basierendes Copolymer als Vormischung und
0 bis 2 Gew.% oberflächenaktive Substanz,
0 bis 25 Gew.% Füllstoff,
0 bis 10 Gew.% Bindemittel,
0 bis 15 Gew.% Sprengmittel,
0 bis 2 Gew.% Schmiermittel und
0 bis 3 Gew.% Gleitmittel,
basierend auf dem Gesamtgewicht der oralen Darreichungsform.

8. Orale Darreichungsform gemäß Anspruch 6 oder 7, wobei die Darreichungsform 270 bis 305 mg Vemurafenib-Cholin oder 540 bis 610 mg Vemurafenib-Cholin umfasst.

9. Orale Darreichungsform gemäß einem der Ansprüche 6 bis 8, wobei die Darreichungsform ein Gesamtgewicht von 400 bis 750 mg oder 800 bis 1450 mg aufweist.

10. Orale Darreichungsform gemäß einem der Ansprüche 6 bis 9, wobei die Inhaltsfreisetzung der Darreichungsform nach 15 Minuten 70% bis 100% ist, wenn gemäß USP-Auflösungsvorrichtung Paddel Typ 2, 900 ml, Phosphatpuffer bei pH 6,8 und 37°C bei 100 Umdrehungen pro Minute gemessen wird.

11. Verfahren zum Herstellen der Vormischung gemäß den Ansprüchen 1 bis 5, umfassend die Schritte
(i) zur Verfügung stellen von Vemurafenib-Cholin oder Vemurafenib als freie Form und kationischem, auf Methacrylaten basierendem Copolymer,
(ii) Mahlen der Mischung von Schritt (i) und
(iii) optionales Sieben der Mischung von Schritt (ii).

12. Verfahren gemäß Anspruch 11, wobei die Bedingungen zum Mahlen in Schritt (ii) so gewählt werden, dass das nach dem Mahlen erhaltende Vemurafenib-Cholin oder Vemurafenib als freie Form in einer nichtkristallinen Form vorliegt.

13. Verfahren zum Herstellen einer oralen Darreichungsform gemäß einem der Ansprüche 6 bis 10, umfassend die Schritte
(i) zur Verfügung stellen von Vemurafenib-Cholin oder Vemurafenib als freie Form und kationischem, auf Methacrylaten basierendem Copolymer,
(ii) Mahlen der Mischung von Schritt (i),
(iii) optionales Sieben der Mischung von Schritt (ii),
(iv) optionales Zugeben von weiteren Hilfsstoff(en) zu der Mischung von Schritt (ii) oder (iii),
(v) optionales Granulieren der Mischung von Schritt (ii), Schritt (iii) oder Schritt (iv) und
(vi) Verarbeiten der Mischung von Schritt (ii), Schritt (iii) oder Schritt (iv) oder den Granulaten von Schritt (v) zu einer oralen Darreichungsform.

14. Orale Darreichungsform gemäß einem der Ansprüche 6 bis 10 zur Verwendung in der Behandlung von Krebs, vorzugsweise Darmkrebs, Ovarialkarzinom, Schilddrüsenkrebs und/oder malignes Melanom.

## Revendications

1. Un prémélange comprenant
a) de la choline de vemurafenib ou du vemurafenib en forme libre, et
b) un copolymère cationique à base de méthacrylates.

2. Le prémélange selon la revendication 1, dans lequel la choline de vemurafenib ou le vemurafenib en forme libre est présent sous une forme non cristalline.

3. Le prémélange selon la revendication 1 ou 2, dans lequel le rapport pondéral entre la choline de vemurafenib et le copolymère cationique à base de méthacrylates est de 1:4 à 4:1.

4. Le prémélange selon la revendication 1 ou 2, dans lequel le rapport pondéral entre le vemurafenib en forme libre et le copolymère cationique à base de méthacrylates est de 1:4 à 4:1.

5. Le prémélange selon l'une quelconque des revendications 1 à 4, dans lequel le pré-mélange a une taille de particule de 0,1 à 100 µm, déterminé au moyen du microscope «Leica DM2500P».

6. Forme posologique orale comprenant le prémélange selon l'une quelconque des revendications 1 à 5.

7. Forme posologique orale selon la revendication 6, dans laquelle la forme posologique comprend
40 à 70% en poids de choline de vemurafenib ou de vemurafenib en forme libre,
25 à 45% en poids d'un copolymère cationique à base de méthacrylates en tant que pré-mélange et
0 à 2% en poids d'agent tensio-actif,
0 à 25% en poids de charge,
0 à 10% en poids de liant,
0 à 15% en poids d'agent de délitement,
0 à 2% en poids de lubrifiant et
0 à 3% en poids d'agent de glissement,
par rapport au poids total de la forme posologique orale.

8. Forme posologique orale selon la revendication 6 ou 7, dans laquelle la forme posologique comprend 270 à 305 mg de choline de vemurafenib ou 540 à 610 mg de choline de vemurafenib.

9. Forme posologique orale selon l'une quelconque des revendications 6 à 8, dans laquelle la forme posologique a un poids total de 400 à 750 mg ou de 800 à 1450 mg.

10. Forme posologique orale selon l'une quelconque des revendications 6 à 9, dans laquelle la libération du contenu de la forme posologique au bout de 15 minutes est de 70% à 100% lorsqu'elle est mesurée conformément à la directive USP, appareil à palettes de type II, 900 ml, tampon phosphate à pH 6,8 et à 37 °C à 100 tours/minute (rpm).

11. Procédé pour la préparation du pré-mélange selon les revendications 1 à 5, comprenant les étapes consistant à
(i) fournir de la choline de vemurafenib ou du vemurafenib en forme libre et copolymère cationique à base de méthacrylates,
(ii) broyer le mélange de l'étape (i) et
(iii) facultativement tamiser le mélange de l'étape (ii).

12. Procédé selon la revendication 11, dans lequel les conditions de broyage à l'étape (ii) sont choisis de telle sorte que la choline de vemurafenib ou le vemurafenib en forme libre est obtenu après broyage sous une forme non cristalline.

13. Procédé de préparation d'une forme posologique orale selon l'une quelconque des revendications 6 à 10, comprenant les étapes consistant à
(i) fournir de la choline de vemurafenib ou du vemurafenib en forme libre et copolymère cationique à base de méthacrylates,
(ii) broyer le mélange de l'étape (i) et
(iii) facultativement tamiser le mélange de l'étape (ii),
(iv) facultativement ajouter un ou plusieurs excipient(s) supplémentaire(s) au mélange de l'étape (ii) ou de l'étape (iii),
(v) facultativement granuler le mélange de l'étape (ii), de l'étape (iii) ou de l'étape (iv), et
(vi) transformer le mélange de l'étape (ii), de l'étape (iii) ou de l'étape (iv) ou les granulés de l'étape (v) en une forme posologique orale.

14. Forme posologique orale selon l'une quelconque des revendications 6 à 10 pour utilisation dans le traitement d'un cancer, de préférence d'un cancer colorectal, d'un cancer de l'ovaire, d'un cancer de la thyroïde et / ou de mélanome malin.
